# EUROPÄISCHE PATENTANMELDUNG

(11) **EP 1 103 619 A1**
(43) Veröffentlichungstag der Anmeldung: **30.05.2001**
(21) Anmeldenummer: 00122041.7
(22) Anmeldetag: 11.10.2000
(51) Int. Cl.: C12P 41/00, C12P 13/04

(54) **Verfahren zur enantioselektiven Gewinnung von Aminosäuren und Aminosäurederivaten unter Verwendung von Racemisierungskatalysatoren**

(30) Priorität: 17.11.1999 DE 19955283
(71) Anmelder: Aventis Research & Technologies GmbH & Co. KG, 65926 Frankfurt am Main (DE)
(72) Erfinder: Riermeier, Thomas, Dr., 65439 Flörsheim (DE); Dingerdissen, Uwe, Dr., 64342 Seeheim-Jugenheim (DE); Gross, Peter, 65451 Kelsterbach (DE); Holla, Wolfgang, Dr., 65779 Kelkheim (DE); Beller, Matthias, Prof. Dr., 18119 Rostock (DE); Schichl, Daniel, Dipl.-Chem., 85748 Garching (DE)
(74) Vertreter: Ackermann, Joachim, Dr.

(57) **Zusammenfassung**

Die Erfindung beschreibt ein Verfahren zur enantioselektiven Gewinnung von Aminosäuren oder Aminosäurederivaten aus deren Estern durch Umsetzung von Gemischen von Enantiomeren- und/oder Diastereomeren des Esters in Gegenwart eines Racemisierungskatalysators der allgemeinen Formel (I), 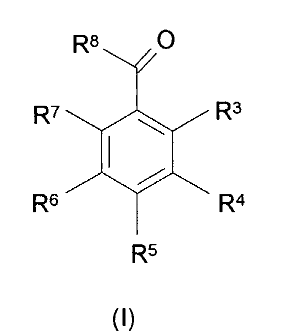 worin R³ eine Gruppe mit einen aciden Proton ist und die Verwendung solcher aromatischer Aldehyde als Racemisierungskatalysator.

## Beschreibung

Die Erfindung beschreibt ein Verfahren zur enzymatisch kinetischen Racematspaltung von Aminosäureestern oder deren Derivaten in Anwesenheit eines Racemisierungskatalysatoren.

Enantiomerenreine Aminosäuren und deren Derivate gehören zu den wichtigsten organischen Verbindungsklassen. Neben der biochemischen Bedeutung sind Aminosäurederivate als Wirkstoffzwischenprodukte, Agrochemikalien, Nahrungsmittelzusatzstoffe sowie als industrielle Feinchemikalien von großem wirtschaftlichem Interesse. Industrielle Herstellverfahren von Aminosäuren beruhen im allgemeinen auf klassischen Methoden, wie der Strecker-Synthese mit nachfolgender Veresterung und Racematspaltung. Der Vorteil derartiger Drei-Stufenverfahren liegt insbesondere im Preis und der Verfügbarkeit der Ausgangsmaterialien sowie der Praktikabilität der einzelnen Reaktionsschritte.

Enantiomer angereicherte Aminosäuren oder deren Derivate werden über eine enantioselektive enzymatische Racematspaltungen verschiedener Aminosäureester mit einer Vielzahl funktionalisierter Aminosäureester in Gegenwart kostengünstiger Enzyme erhalten. Zusammenstellungen findet man beispielsweise in M. A. Verkhovskaya, I. A. Yamskov, *Russ. Chem. Rev.* 1991, *60*, 1163 sowie H. Stecher, K. Faber, *Synthesis,* 1997, 1. Nachteilig sind bei diesen Racematspaltungsverfahren jedoch die ungenügende Atomökonomie des Racematspaltungsschrittes (max. 50 % Ausbeute bei Batchverfahren) und die aufwendige Abtrennung des unerwünschten Aminosäureesters mit anschließender Racemisierung.

Die Problematik einer enantiomerenreinen Synthese von Aminosäuren kann an [L]-2-Amino-4-methylphosphinobuttersäure (L-Ptc) exemplarisch erläutert werden. (L-Ptc) ist Bestandteil eines literaturbekannten, mikrobiell gewonnenen Antibiotikums (DE 22 36 599) und kann daraus durch Hydrolyse gewonnen werden. Die mangelnde Effizienz dieser Fermentation macht eine chemische Synthese von Ptc notwendig. Während der racemische Wirkstoff leicht zugänglich ist , ist enantiomerangereichertes L-Ptc nur vergleichsweise aufwendig darstellbar.

Da die bekannten Syntheserouten für L-Ptc technisch nicht praktikabel sind, wurden Racematspaltungen von DL-Ptc entwickelt. So sind etliche enzymatische Deacylierungen von N-acylierten DL-Ptc-Derivaten beschrieben (US 4,2262941; EP 358 428; EP 342 575; EP 301 391; EP 054 897; EP 382 113). Darüber hinaus ist die kinetische Racematspaltung der entsprechenden Amide von DL-Ptc bekannt (EP 690 133). Die beschriebenen kinetischen Racematspaltungsreaktionen haben gravierende Nachteile, da bei ihnen der Umsatz auf 50 % beschränkt ist, muß das Reaktionsprodukt aufwendig vom nicht umgesetzten Edukt getrennt werden. Im Falle des Ptc ist dies aufgrund der äußerst ähnlichen physikochemischen Eigenschaften sehr aufwendig. Desweiteren muß in einen zweiten Verfahrensschritt das wiedergewonnene, erneut einsetzbare Edukt zuvor racemisiert werden.

Vor diesem Hintergrund sind dynamische Racematspaltungen bei denen das unerwünschte Enantiomer des Aminosäureesters unter den Reaktionsbedingungen racemisiert, so daß die Ausgangsmaterialien fast vollständig in enantiomerenreine Aminosäuren überführt werden können, von großem Interesse.

Auguste und Mitarbeiter (EP 0 089 886, 1983) bzw. Chen und Mitarbeiter (*J. Org. Chem.* 1994, *59*, 7580) beschrieben die Racemisierung von Aminosäureestern in Gegenwart von Enzymen mit Pyridoxal-5-phosphat als Katalysator. Obwohl hier erstmals gezeigt wurde, daß dynamische Racematspaltungen von Aminosäureestern möglich sind, sind die Nachteile dieser Variante der außerordentlich hohe Preis des Katalysators und die große Menge an Pyridoxal-5-phosphat - üblicherweise 20 mol-% bis 100 mol-% -, die notwendig ist, entsprechende Aminosäureester zu racemisieren. Wirtschaftlich gesehen sind sogar einfache Racematspaltungen kostengünstiger als die von Chen et al. beschriebene Variante.

Die Verwendung kostengünstigerer Aldehyde gelingt laut Literatur (Parmar et al., *J. Org. Chem.* 1996, *61*, 1223) nur dann in befriedigenden Ausbeuten, wenn in einer zusätzlichen Reaktionsstufe mit großen, d. h. zumindest stöchiometrischen Mengen eine Schiff-Base erzeugt wird, welche dann der enzymatischen Hydrolyse unterworfen wird.

Aus den genannten Gründen besteht ein großer Bedarf an neuen Katalysatorsystemen, die kostengünstig sind und die Aminosäureester unter den Bedingungen der enzymatischen Racematspaltung racemisieren, so daß die Aminosäure in enantiomer angereicherter Form in hoher Ausbeute und Reinheit bei geringem Katalysatoreinsatz gewonnen werden kann.

Das beanspruchte Verfahren erfüllt diese Anforderungen durch eine stereodifferenzierende enzymatische Esterspaltung von Aminosäure-Carbonsäureestern oder Carbonsäureester von Aminosäurederivaten unter gleichzeitiger Racemisierung des Esters, in Gegenwart geringer Mengen eines einfachen Racemisierungsakatalysators. Dabei wurde überraschenderweise gefunden, daß unter den gewählten Bedingungen der Carbonsäureester selektiv in Gegenwart von gelösten L- oder D-Aminosäuren racemisiert werden kann. Das Produkt wird in enantiomeren angereicherter Form in Ausbeuten von über 50 % erhalten.

Gegenstand der vorliegenden Erfindung ist somit die Bereitstellung eines neuen Verfahrens zur Herstellung von enantiomeren angereicherten L- oder D-Aminosäuren und deren Derivaten bzw. deren Vorstufen, welche nach einfachen Standardmethoden in L- oder D-Aminosäuren überführt werden können. Ein weiterer Gegenstand der Erfindung ist die Verwendung von aromatischen Aldehyden gemäß Anspruch 1 als Racemisierungskatalysatoren.

Das Verfahren zur enantioselektiven Gewinnung von Aminosäuren oder Aminosäurederivaten aus deren Estern erfolgt durch Umsetzung von Gemischen aus Enantiomeren- und/oder Diastereomeren des Esters in Gegenwart eines Racemisierungskatalysators der Formel (I), 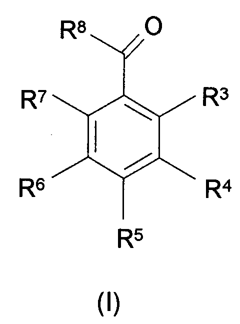 wobei R³ eine Gruppe mit einen aciden Proton, bevorzugt mit einem pKₛ-Wert zwischen 2 und 7, beispielsweise aus der Gruppe COOH, OH, SH, SO₃H, B(OH)₂, NHCOCH₃ oder PO(OH)₂ ist,
R⁴-R⁷ unabhängig voneinander Wasserstoff, Alkyl, O-Alkyl, COO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NArylAlkyl, NHCO-Aryl und
R⁸ Wasserstoff, Alkyl oder Aryl bedeuten,
wobei Alkyl eine aliphatische Kohlenstoffgruppe mit 1 bis 18 C-Atomen, die linearen, verzweigt, gesättigt oder ungesättigt und/oder auch cyclisch ist, bedeutet und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet, dabei können sowohl die Alkyl- als auch die Arylgruppe bis zu sechs weitere Substituenten tragen, die unabhängig voneinander Wasserstoff, Alkyl, O-Alkyl, COO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NarylAlkyl, NHCO-Aryl bedeuten, wobei Alkyl und Aryl die oben genannte Bedeutung haben, dabei können R⁷ und R⁸ auch durch eine kovalente Bindung verknüpft sein, so daß ein anneliertes Ringsystem vorliegt,

in homogenen oder heterogenen, wäßrigen, wäßrig-organischen oder organischen Medien in Gegenwart eines gegebenenfalls immobilisierten Enzyms zur enantioselektiven Esterspaltung, bevorzugt einer stereoselektiven Hydrolyse oder Alkoholyse, von Verbindungen der Formel (II), z. B. einer Lipase oder Esterase, z. B. aus Säugetier-Lebern oder Bauchspeicheldrüsen oder mikrobiellen Ursprungs, wie beispielsweise aus Candida, Pseudomonas und Aspergillus, oder einer Proteasen, z. B. aus Bacillus, bei einer Temperatur von 10 - 100 °C gegebenenfalls in Gegenwart von Cosolventien und eines Puffers, wobei die Reaktionsmischung vorzugsweise 2 - 50 Gew.-% Aminosäureester enthält.

Das erfindungsgemäße Verfahren ist ökonomisch, einfach und schnell durchführbar. Die Reaktion erfordert keine äquimolaren Mengen optisch reiner Hilfsstoffe, keine teuren, schwer zugänglichen Reagenzien, keine unverhältnismäßig großen Lösungsmittelmengen und keine aufwendigen Arbeitsschritte. Nach Beendigung der Reaktion wird das enantiomer angereicherte Produkt durch einfache Maßnahmen, z. B. durch Extraktion oder Kristallisation endgereinigt.

Bevorzugt sind Racemisierungskatalysatoren der Formel (I), wobei
R³ für OH oder COOH, R⁴ bis R⁷ unabhängig voneinander für Wasserstoff, Alkyl, O-Alkyl, O-Aryl, Aryl, Fluor, Chlor, Brom, NO₂, CN, CHO, SO₃H, PO-Alkyl₂, SO₂-Alkyl, CF₃, NHCO-Alkyl, CONH₂, CO-Alkyl, CO-Aryl, COO-Aryl, PO₃H₂, SO₃-Alkyl und R⁸ Wasserstoff, Alkyl oder Aryl stehen, dabei können R⁷ und R⁸ auch durch eine kovalente Bindung verknüpft sein, so daß ein anneliertes Ringsystem vorliegt, wie beispielsweise in substituierten Indanonen oder Fluorenonen, steht.

Besonders bevorzugt sind als Racemisierungskatalysatoren folgende Verbindungen der Formel (I) worin :
R³ für OH, R⁴ bis R⁷ unabhängig voneinander für Wasserstoff, Fluor, Chlor, Brom, NO₂, CN, CHO, SO₃H, CF₃, CO-Alkyl, CO-Aryl, und R⁸ Wasserstoff,
steht.

Mit Hilfe elektronenziehender Substituenten kann die katalytische Aktivität des Restes R³ des Racemisierungskatalysators gesteigert werden. Folglich sind die Verbindungen der Formel (I) mit mindestens einem elektronenziehenden Substituenten, insbesondere mit einem OCO-Alkyl, Fluor, Chlor, Brom, lod, OH, NO₂, NO, CN, COOH, CHO, SO₃H, CF₃, CO-Alkyl, CO-Aryl, COO-Aryl, POAryl₂, SO₃-Alkyl Substituenten aus der Gruppe der Reste R⁴ bis R⁷ bevorzugt. Besonders bevorzugt ist Rest R⁴ und/oder R⁶ ein solcher Substituent. Mit Hilfe von elektronenspendenden Substituenten an den freien Positionen des aromatischen Rings, bevorzugt als Rest R⁵ und/oder R⁷, kann die Stabilität der Katalysator-Substrat-Verbindung und damit die Reaktionsgeschwindigkeit und der Katalysatorverbrauch gesenkt werden. Die einfache und leichte Anpassungsfähigkeit von Racemisierungskatalysatoren der Formel (I) über dessen Substitutionsmuster auf unterschiedliche Reaktionsbedingungen, insbesondere auf unterschiedliche Substrate ist ein weiterer Vorteil der vorliegenden Erfindung. Zusätzlich kann über die Substituenten die Löslichkeit in den verschiedenen Medien verbessert werden.

Als Substrat werden bevorzugt Verbindungen der Formel (II), 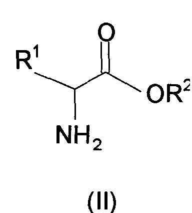 worin R¹und R² unabhängig voneinander für einen Alkyl-, Alkenyl-, Alkinyl-, Aryloder Heteroarylrest steht, eingesetzt, wobei Alkyl-, Alkenyl-, Alkinyl- eine aliphatische Kohlenstoffgruppe mit 1 bis 18 C-Atomen, die linearen, verzweigt und/oder auch cyclisch ist, stehen und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet, dabei können sowohl die Alkyl- als auch die Arylgruppe gegebenenfalls bis zu sechs weitere Substituenten tragen, die unabhängig voneinander Wasserstoff, Alkyl, O-Alkyl, OCO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, S-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, PO(O-Aryl)₂, PO(O-Alkyl)(O-Aryl), PO(O-Alkyl)Alkyl, PO(O-Alkyl)Aryl, PO(O-Aryl)Alkyl, PO₂H-Alkyl, PO₂H-Aryl, PO(OH)Alkyl, PO(OH)Aryl SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NArylAlkyl, NHCO-Aryl bedeuten und R¹auch für CH₂CH₂POAryl₂, CH₂CH₂PO₃H₂, CH₂CH₂PO(O-Alkyl)₂, CH₂CH₂PO(O-Aryl)₂, CH₂CH₂PO(O-Alkyl)(O-Aryl), CH₂CH₂PO(O-Alkyl)Alkyl, CH₂CH₂PO(O-Alkyl)Aryl, CH₂CH₂PO(O-Aryl)Alkyl, CH₂CH₂PO₂H-Alkyl, CH₂CH₂PO₂H-Aryl, stehen kann, wobei Alkyl und Aryl die oben genannte Bedeutung haben.

Bevorzugt stehen in den Verbindungen der Formel (II)
R¹ für CH₂CH₂POAryl₂, CH₂CH₂PO₃H₂, CH₂CH₂PO(O-Alkyl)₂, CH₂CH₂PO(O-Aryl)₂, CH₂CH₂PO(O-Alkyl)(O-Aryl), CH₂CH₂PO(O-Alkyl)Alkyl, CH₂CH₂PO(O-Alkyl)Aryl, CH₂CH₂PO(O-Aryl)Alkyl, CH₂CH₂PO₂H-Alkyl, CH₂CH₂PO₂H-Aryl, (C₄-C₁₄)-Aryl, (C₁-C₁₈)-Alkyl, (C₂-C₁₈)-Alkenyl bzw. (C₃-C₁₈)-Alkinyl, CₙH₂ₙ-Cycloalkyl mit n = 1-18, die verzweigt oder nichtverzweigt sein können und die substituiert sein können mit 1-6 Substituenten, die unabhängig voneinander Wasserstoff, Alkyl, O-Alkyl, O-Aryl, Aryl, Fluor, Chlor, Brom, lod, OH, NO₂, CN, COOH, CHO, NH₂, NH-Alkyl, N-Alkyl₂, PO-Alkyl₂, SO₂-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂ steht und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann und
R² für (C₄-C₁₄)-Aryl, (C₄-C₁₄)-Aryl, (C₁-C₁₂)-Alkyl, (C₂-C₁₂)-Alkenyl bzw. (C₃-C₁₂)-Alkinyl, CₙH₂ₙ-Cycloalkyl mit n = 1-12, die verzweigt oder nichtverzweigt sein können und die substituiert sein können mit 1-3 Substituenten, die unabhängig voneinander Aryl, Fluor, Chlor, Brom, lod, NO₂, CN, CF₃, Methoxy und/oder COOR⁹, mit R⁹ = Methyl oder Ethyl, die substituiert sein können mit Substituenten aus der Gruppe Fluor, Chlor, Brom, lod, CF₃ und/oder Methoxy steht.

Besonders bevorzugt steht in den Verbindungen der Formel (II)
R¹für CH₂CH₂PO(O-Alkyl)Alkyl, CH₂CH₂PO(O-Alkyl)Aryl, CH₂CH₂PO(O-Aryl)Alkyl, CH₂CH₂PO₂H-Alkyl, CH₂CH₂PO₂H-Aryl, (C₅-C₁₂)-Aryl, (C₁-C₁₆)-Alkyl, (C₂-C₁₆)-Alkenyl bzw. (C₃-C₁₆)-Alkinyl, CₙH₂ₙ-Cycloalkyl mit n = 1-16, die verzweigt oder nichtverzweigt sein können und die substituiert sein können mit 1-4 Substituenten, die unabhängig voneinander Wasserstoff, Alkyl, O-Alkyl, O-Aryl, Aryl, Fluor, Chlor, Brom, lod, OH, NO₂, CN, COOH, CHO, NH₂, NH-Alkyl, N-Alkyl₂, CF₃, NHCO-Alkyl, CONH₂, CO-Alkyl, NHCOH, NHCOO-Alkyl, CO-Aryl, COO-Aryl, steht und Aryl einen 4 bis zu 12 C-Atome enthaltenden fünf-, sechs- oder siebengliedrigen aromatischen Ring, wobei dieser Ring anneliert sein kann und 0 bis 3 Heteroatome wie N, O, S enthalten kann und
R² für (C₄-C₁₀)-Aryl, (C₁-C₁₀)-Alkyl, (C₂-C₁₀)-Alkenyl bzw. (C₃-C₁₀)-Alkinyl, CₙH₂ₙ-Cycloalkyl mit n = 1-10, die verzweigt oder nichtverzweigt sein können und die substituiert sein können mit 1-3 Substituenten, die unabhängig voneinander Aryl, Fluor, Chlor, Brom, lod, NO₂, CN, CF₃, Methoxy und/oder COOR⁹, mit R⁹ = Methyl oder Ethyl, die substituiert sein können mit Substituenten aus der Gruppe Fluor, Chlor, Brom, lod, CF₃ und/oder Methoxy steht.

Ganz besonders bevorzugt steht in den Verbindungen der Formel (II)
R¹ für CH₂CH₂PO(O-Alkyl)CH₃, CH₂CH₂PO(O-Aryl)CH₃, CH₂CH₂PO₂H-CH₃, (C₅-C₁₀)-Aryl, (C₁-C₁₄)-Alkyl, (C₂-C₁₄)-Alkenyl bzw. (C₃-C₁₄)-Alkinyl, CₙH₂ₙ-Cycloalkyl mit n = 1-14, die verzweigt oder nichtverzweigt sein können und die substituiert sein können mit 1-3 Substituenten, die unabhängig voneinander Wasserstoff, Alkyl O-Alkyl, O-Aryl, Aryl, Fluor, Chlor, Brom, lod, OH, NO₂, CN, COOH, CHO, NH₂, NH-Alkyl, N-Alkyl₂, CF₃, NHCO-Alkyl, CONH₂, CO-Alkyl, CO-Aryl, COO-Aryl, steht und Aryl einen 4 bis zu 10 C-Atome enthaltenden fünf-, sechs- oder siebengliedrigen aromatischen Ring, wobei dieser Ring anneliert sein kann und 0 bis 3 Heteroatome wie N, O, S enthalten kann und
R² für (C₄-C₁₀)-Aryl, (C₁-C₁₀)-Alkyl, (C₂-C₁₀)-Alkenyl bzw. (C₃-C₁₀)-Alkinyl, CₙH₂ₙ-Cycloalkyl mit n = 1-10, die verzweigt oder nichtverzweigt sein können und die substituiert sein können mit 1-3 Substituenten, die unabhängig voneinander Phenyl, Fluor, Chlor, Brom, lod, NO₂, CN, CF₃, Methoxy und/oder COOR⁹, mit R⁹ = Methyl oder Ethyl steht.

Bei dem Verfahren wird vorzugsweise wie folgt vorgegangen. Ein Ester der Formel (II), beispielsweise R² = CH₃ bis C₈H₁₇ wird in einer wasser- oder alkoholhaltigen Lösung mit einer Lipase, Esterase oder Protease versetzt und gerührt.

Es ist vorteilhaft die genannte Lösung zu puffern, z. B. mit Phosphat- oder TRIS [= Tris-(hydroxymethyl)-methylamin]-Puffer oder eine Base wie beispielsweise Ammoniak oder Natronlauge kontinuierlich zuzuführen. Ein günstiger Pufferbereich ist pH 6,5 - 9,5. Die Neutralisationsbedingungen können so gewählt werden, daß direkt die gewünschten Zwischen- oder Endprodukte erhalten werden. Wird z. B. mit Ammoniak neutralisiert, kann z. B. Verbindung III b) (siehe unten) direkt erhalten werden.

Es kann weiterhin vorteilhaft sein, Cosolventien zuzugeben. Als Cosolvens eignen sich z. B. Dimethoxyethan, Aceton, Acetonitril, THF, Dioxan, Hexan, tert.-Butylmethylether und tert.-Butanol. Der Anteil an Cosolvens in der Lösung liegt vorzugsweise bei 10 - 90 %.

Als Enzyme werden bevorzugt Lipasen und Proteasen, wie z. B. α-Chymotrypsin (EC 3.4.21.1 aus Rinderpankreas (Fluka Chemie AG)), Alcalase (EC 3.4.21.14 Protease von *bacillus licheniformis ,* Novo Industri A/S Dänemark)), Subtilisin (EC 3.4.21.62 Protease von *Bacillus licheniformis* (Fluka Chemie AG); Chirazym P1 (Roche Diagnostics); Chiroclec® -BL (Altus Biologics)) und Lipase aus Schweinepankreas (PPL, EC 3.1.1.3 (Sigma Chemical Co.)) eingesetzt.

Jedes der genannten Enzyme kann in freier oder in immobilisierter Form (Immobilized Biocatalysts, W. Hartmeier, Springer Verlag Berlin, 1988) eingesetzt werden. Die Enzymmenge wird in Abhängigkeit von der Reaktionsgeschwindigkeit bzw. von der angestrebten Reaktionszeit und von der Art des Enzyms (z. B. frei oder immobilisiert) frei gewählt und ist durch einfache Vorversuche leicht zu bestimmen.

Die Reaktionsmischung enthält bezogen auf das Lösemittel vorzugsweise 2-50 Gew.-% Ester, besonders bevorzugt 5-20 Gew.-%. Die Reaktionstemperatur liegt zwischen 0 °C und 100 °C, bevorzugt zwischen 20 °C und 80 °C, besonders bevorzugt zwischen 20 °C und 60 °C.

Die Herstellung der Carbonsäureester erfolgt zweckmäßigerweise aus der Aminosäure oder deren Derivaten nach bekannten Methoden der Veresterung (Haslam, Tetrahedron 1980, 36, 2409; Höfle, Steglich, Vorbrüggen, Angew. Chem. 1978, 90, 602).

Nach dem erfindungsgemäßen Verfahren wird der Racemisierungskatalysator in katalytischen Mengen bezogen auf das Substrat, also den Aminosäureester oder einem Aminosäureesterderivat eingesetzt. Generell werden zwischen 20mol% und 0.01 mol% bezogen auf das Substrat, bevorzugt 15 bis 0.1 mol% und besonders bevorzugt 10 bis 0.1 mol% verwendet.

Ein Anwendungsbeispiel des erfindungsgemäßes Verfahrens ist die enantioselektive Herstellung von [L]-2-Amino-4-methylphosphinobuttersäure (L-Phosphinothricin, L-Ptc) bzw. dessen Ammoniumsalz. Herkömmlich werden nahezu racemische Mischungen aus DL-Ptc bzw. dessen Ammoniumsalz als Herbizid eingesetzt. Die herbizide Wirkung entfalten dabei vorallem L-Form (L-llla; L-Phosphinothricin; L-Ptc bzw. dessen Ammoniumsalz L-lllb), während die jeweilige enantiomere D-Form nahezu unwirksam ist.

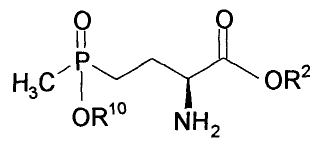

L-IIIa : R¹⁰ = H; R² = H

L-IIIb : R¹⁰ = NH₄; R² = H

Da also unter Verwendung herkömmlicher industrieller Herstellungsmethoden etwa die Hälfte des eingesetzten Phosphinothricins kaum herbizide Wirkung zeigt, lassen sich durch den Einsatz von enantiomer angereichertem L-Ptc somit geringere Aufwandmengen, geringere Formulierungshilfsmittel und weniger an im Boden und in den Pflanzen biologisch abzubauende Stoffe realisieren. Die Verwendung von enantiomer angereichertem L-Ptc ist somit von großem ökologischen und ökonomischen Interesse.

Durch die nachfolgenden Ausführungsbeispiele soll die vorliegende Erfindung näher erläutert werden, ohne sie darauf zu beschränken.

### Beispiele:

Die Produkte wurden durch ¹H-NMR-Spektren identifiziert. Die optische Reinheit der Produkte wurde durch Messung des spezifischen Drehwertes und über HPLC-Analyse bestimmt.

### Beispiel 1:

20 mmol racemisches Tyrosinesterhydrochlorid (Verbindung der Formell (II) mit R¹= 4-OH-C₆H₄ und R² = CH₂C₆H₅ bzw. C₂H₅) werden in einem Gemisch aus 10 mL H₂O und 40 mL Acetonitril gelöst und mit 2 normaler NaOH auf den gewünschten pH-Wert eingestellt. Die dabei entstehende wäßrige Phase wird abgetrennt und verworfen. Der Reaktionsansatz wird an einen Autotitrator angeschlossen und auf Reaktionstemperatur gebracht. Durch die Zugabe von 0.2 mL Alcalase (Protease von *bacillus lichenifomis;* Aktivität: 2,5 AU/mL) und der gewählten Aldehydmenge (siehe Tabelle 1) wird die Reaktion gestartet. Nach Erreichen von mindestens 90 % Umsatz (HPLC) wird der Reaktionsansatz auf pH 5.5-6.5 eingestellt, auf 0 °C gekühlt und eine Stunde gerührt. Das ausfallende Produkt wird abfiltriert und mit 50 - 200 mL Acetonitril gewaschen.

**Tabelle 1**

| Substrat | Reaktions-temperatur | Reaktionszeit in Tagen | pH-Wert | Aldehyd | Ausbeute in [%] | ee(L) in [%] |
|---|---|---|---|---|---|---|
| D,L-TyrOBzl | 35°C | 2 | 8,5 | 5mol% B | 92 | 97,0 |
| D,L-TyrOBzl | 35°C | 1 | 8,5 | 5mol% A | 95 | 99,2 |
| D,L-TyrOBzl | 35°C | 1 | 8,5 | 5mol% B | 87 | 96,8 |
| D,L-TyrOEt | 35°C | 3 | 7,5 | 5mol% B | 92 | 98,0 |
| D,L-TyrOEt | Raum-temperatur | 0,8 | 8,0 | 5mol% B | 85 | 97,7 |
| A: 3,5-Dinitrosalicylaldehyd B: 3,5-Dichlorsalicylaldehyd | | | | | | |

NMR-spektroskopische Charakterisierung von Tyrosin ¹H-NMR (200 MHz Trifluoressigsäure)
δ= 3,5 (ddd/2 dd, br, ³J = 15,4 Hz, ³J = 8,6 Hz, ³J = 4,6 Hz, C*H*₂); 4,6 (dd, ³J = 8,5 Hz, ³J = 4,9 Hz, 1H, C*H*); 7,1 (d, ³J = 8,6 Hz, 2H, *H*_{*arom*}*);* 7,3 (d, ³J = 8,3 Hz, 2H, *H*_{*arom*}*);* 11,6 (s, br, 1 H, CO₂*H*).

### Beispiel 2 :

20 mmol racemisches Aminosäureesterhydrochlorid (Verbindung der Formell (II) mit R¹= C₆H₄ und R² = CH₂C₆H₅) werden in einem Gemisch aus 10 mL H₂O und 40 mL Acetonitril gelöst und mit 2 normaler NaOH auf den pH 8.5 eingestellt. Die dabei entstehende wäßrige Phase wird abgetrennt und verworfen. Der Reaktionsansatz wird an einen Autotitrator angeschlossen und auf Reaktionstemperatur gebracht. Durch die Zugabe von 0.2 mL Alcalase (Protease von *bacillus lichenifomis;* Aktivität: 2,5 AU/mL) und 10 mol% Aldehyd (Tabelle 2) wird die Reaktion gestartet. Nach Umsatzstillstand (HPLC) wird der Reaktionsansatz mit 100 mL Dimethoxyethan versetzt und auf pH 5.5-6.5 eingestellt, auf 0 °C gekühlt und eine Stunde gerührt. Das ausfallende Produkt wird abfiltriert und mit 50 - 200 mL Acetonitril gewaschen.

**Tabelle 2**

| Reaktions-Temperatur | Reaktionszeit in Tagen | Aldehyd | Ausbeute in [%] | Ee(L) in [%] |
|---|---|---|---|---|
| 35°C | 1 | 10mol% B | 71 | 83 |
| 35°C | 1 | 10mol% A | 79 | 70 |
| 35°C | 1 | 10mol% B | 87 | 88 |
| A: 3,5-Dinitrosalicylaldehyd B: 3,5-Dichlorsalicylaldehyd | | | | |

NMR-spektroskopische Charakterisierung von Phenylalanin ¹H-NMR (200 MHz Trifluoressigsäure)
δ= 3,5 (ddd/2 dd, ³J = 8,8 Hz, ³J = 4,8 Hz, ³J = 14,9 Hz, C*H*₂); 4,7 (dd, ³J = 8,4 Hz, ³J = 4,8 Hz, 1H, C*H*); 7,3-7,5 (m, 5H, 2H, C*H*_{*arom*}*);* 11,5 (s, br, 1H, CO₂*H*).

### Beispiel 3:

20 mmol racemisches Aminosäureesterhydrochlorid (Verbindung der Formell (II) mit R¹= C₆H₄ und R² = C₂H₅ und C₄H₉) werden in einem Gemisch aus 10 mL H₂O und 40 mL Acetonitril gelöst und mit 2 normaler NaOH auf den pH 7.5 eingestellt. Die dabei entstehende wäßrige Phase wird abgetrennt und verworfen. Der Reaktionsansatz wird an einen Autotitrator angeschlossen und auf Reaktionstemperatur gebracht. Durch die Zugabe von 0.2 mL Alcalase (Protease von *bacillus lichenifomis;* Aktivität: 2,5 AU/mL) und 5 mol% 3,5-Dichlorsalicylaldehyd wird die Reaktion gestartet. Nach Umsatzstillstand (HPLC) wird der Reaktionsansatz mit 100 mL Dimethoxyethan versetzt und auf pH 5.5-6.5 eingestellt, auf 0 °C gekühlt und eine Stunde gerührt. Das ausfallende Produkt wird abfiltriert und mit 50 - 200 mL Acetonitril gewaschen.

**Tabelle 3**

| Substrat | Reaktionszeit in Tagen | Ausbeute in [%] | ee(L) in [%] |
|---|---|---|---|
| D,L-PheOEt | 5 | 72 | 98,3 |
| D,L-PheOnBu | 7 | 63 | 92,3 |

NMR-spektroskopische Charakterisierung von Phenylalanin ¹H-NMR (200 MHz Trifluoressigsäure)
δ = 3,5 (ddd/2 dd, ³J = 8,8 Hz, ³J = 4,8 Hz, ³J = 14,9 Hz, C*H*₂); 4,7 (dd, ³J = 8,4 Hz, ³J = 4,8 Hz, 1 H, C*H*); 7,3-7,5 (m, 5H, 2H, C*H*_{*arom*}*);* 11,5 (s, br, 1 H, CO₂*H*).

### Beispiel 4:

Zu 50 mL Wasser werden bei pH 7.5 unter rühren 1 mL (Protease von *bacillus lichenifomis;* Aktivität: 0,6 AU/mL) und 2,5 mol% 3,5-Dichlorsalicylaldehyd gegeben. Die Reaktion wird durch langsames Zutropfen einer Lösung aus 20 mmol racemischen Aminosäureester (Verbindung der Formell (II) mit R¹= C₆H₄, C₂H₅ CH(CH₃)₂, CH₂SCH₃ und R² = C₂H₅) in 50 mL Acetonitril gestartet. Der pH-Wert der Lösung wird mit einem Autotitrator auf pH 7.5 eingestellt.

Die in Tabelle 4 erhaltenen Daten über den Umsatz und Enantiomerenreinheit wurden mit GC- bzw. HPLC-Methoden ermittelt.

**Tabelle 4:**

| Substrat | Reaktionszeit in Tagen | Ausbeute in [%] | ee(L) in [%] |
|---|---|---|---|
| D,L-PheOEt | 3 | 96 | 95 |
| D,L-MetOMe | 3 | 96 | 89 |
| D, L-NvalOEt | 4 | 98 | 60 |
| D,L-LeuOEt | 4 | 99 | 98 |

Beispiele zur Herstellung von Verbindungen der Formel (III):

Alle Produkte wurden durch ¹H-und ³¹P-NMR-Spektren und einer Schmelzpunktbestimmung identifiziert. Die optische Reinheit der Produkte wurde durch Messung des spezifischen Drehwertes und über HPLC-Analyse (EP 0 382 113) bestimmt.

Aufarbeitung: Nach Entfernen des Lösemittels kann Verbindung Illb direkt durch Umkristallisation aus Methanol erhalten werden.

Das Hydrochlorid von la kann durch die Behandlung der methanolischen Lösung mit 6N HCI erhalten werden. Auf diese Weise wird auch der racemische Ester (II) hydrolysiert, so daß auch bei nicht vollständigen Umsatz die isolierte Ausbeute von Ia^{∗}HCI 90 % - 95 % beträgt.

### Beispiele 5 - 8 :

In einem Vierhalskolben mit automatischer pH- und Temperatur-Kontrolle werden 42 mmol Phosphinothricin-n-butylester und 0,47 mmol 3,5-Dinitrosalicylaldehyd in einer Mischung eines organischen Lösemittel und Wasser (Mischverhältnisse vgl. Tabelle) gelöst. Nach dem Einstellen des pH auf 7 mit 12%iger wäßriger Ammoniaklösung werden 100 mg Chirazym P-1.lyo (Roche Diagnostics) zugesetzt. Die Reaktionslösung wird die angegebene Zeit bei 35 - 38 °C gerührt. Durch oben beschriebene Aufarbeitung erhält man IIIa^{∗}HCI mit den angegebenen Enantiomerenüberschüssen.

**Tabelle 5**

| Beispiel | Lösemittel (ml) | Wasser (ml) | Reaktionszeit in Stunden | ee(L) in [%] |
|---|---|---|---|---|
| 5 | Acetonitril (40) | 15 | 20 | 45 |
| 6 | t-Butanol (10) | 40 | 6 | 57 |
| 7 | t-Butanol (40) | 10 | 20 | 62 |
| 8 | --- | 50 | 30 | 18 |

### Beispiel 9:

In einem Vierhalskolben mit automatischer pH- und Temperatur-Kontrolle werden 42 mmol Phosphinothricin-n-butylester und 0,6 mmol 3,5-Dinitrosalicylaldehyd in einer Mischung aus 50 ml Acetonitril und 10 ml Wasser gelöst. Nach dem Einstellen des pH auf 7 mit 12%iger wäßriger Ammoniaklösung werden 50 mg Chirazym P-1.lyo (Roche Diagnostics) zugesetzt. Die Reaktionslösung wird 20 h bei 35 - 38 °C gerührt. Durch die oben beschriebene Aufarbeitung erhält man IIIa^{∗}HCI mit einem Enantiomerenüberschuß von 72 %.

### Beispiele 10 - 13:

In einem Vierhalskolben mit automatischer pH- und Temperatur-Kontrolle werden 21 mmol Phosphinothricin-n-butylester und 0,47 mmol 3,5-Dinitrosalicylaldehyd in einer Mischung eines organischen Lösemittel und Wasser (Mischverhältnisse vgl. Tabelle) gelöst. Nach dem Einstellen des pH auf 7 mit 12%iger wäßriger Ammoniaklösung werden 150 mg Chirazym P-1.lyo (Roche Diagnostics) zugesetzt. Die Reaktionslösung wird die angegebene Zeit bei 28 - 31 °C gerührt. Durch oben beschriebene Aufarbeitung erhält man IIIa^{∗}HCI mit den angegebenen Enantiomerenüberschüssen.

**Tabelle 6**

| Beispiel | Lösemittel (ml) | Wasser (ml) | Reaktionszeit in Stunden | ee(L) in [%] |
|---|---|---|---|---|
| 10 | t-Butanol (50) | 10 | 20 | 75 |
| | Methyl-t-butylether (20) | | | |
| 11 | t-Butanol (40) | 10 | 20 | 69 |
| 12 | ---- | 50 | 7 | 46 |
| 13 | Acetonitril (50) | 12 | 8^{∗} | 43 |

### Beispiel 14:

In einem Vierhalskolben mit automatischer pH- und Temperatur-Kontrolle werden 21 mmol Phosphinothricin-n-butylester und 0,47 mmol 3,5-Dinitrosalicylaldehyd in einer Mischung aus 40 ml Acetonitril und 5 ml Wasser gelöst. Nach dem Einstellen des pH auf 7 mit 12%iger wäßriger Ammoniaklösung werden 1,8 ml ChiroClec-BL (Altus Biologics Inc.) zugesetzt. Die Reaktionslösung wird 20 h bei 40 - 45 °C gerührt. Durch die oben beschriebene Aufarbeitung erhält man IIIa^{∗}HCI mit einem Enantiomerenüberschuß von 48 %.

### Beispiel 15:

In einem Vierhalskolben mit automatischer pH- und Temperatur-Kontrolle werden 21 mmol Phosphinothricin-n-butylester und 1,41 mmol 3,5-Dinitrosalicylaldehyd in einer Mischung aus 50 ml Acetonitril und 15 ml Wasser gelöst. Nach dem Einstellen des pH auf 7 mit 12%iger wäßriger Ammoniaklösung werden 100mg Chirazym P-1.lyo zugesetzt. Die Reaktionslösung wird 50 h bei 30 - 32 °C gerührt. Durch die oben beschriebene Aufarbeitung erhält man IIIa^{∗}HCI mit einem Enantiomerenüberschuß von 83 %.

## Patentansprüche

1. Verwendung von Verbindungen der Formel (I) als Racemisierungskatalysator zur enantioselektiven Gewinnung von Aminosäuren und Aminosäurederivaten durch dynamisch kinetische Racematspaltung, 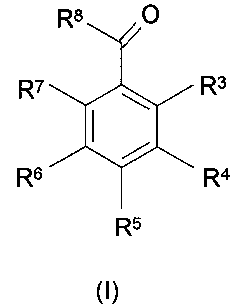 worin
R³ eine Gruppe mit einen aciden Proton ist,
R⁴-R⁷ unabhängig voneinander Wasserstoff, Alkyl, O-Alkyl, COO-Alkyl, Aryl, O-Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, NHCOH, NHCOO-Alkyl, CO-Aryl, COO-Aryl, PO-Aryl₂, PO₃H₂, PO(O-Alkyl)₂, SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NarylAlkyl, NHCO-Aryl sind und
R⁸ Wasserstoff, Alkyl oder Aryl ist, und
wobei Alkyl eine aliphatische Kohlenstoffgruppe mit 1 bis 18 C-Atomen, die linearen, verzweigt, gesättigt oder ungesättigt und/oder auch cyclisch ist, bedeutet und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet, dabei können sowohl die Alkyl- als auch die Arylgruppe bis zu sechs weitere Substituenten tragen, die unabhängig voneinander Wasserstoff, Alkyl, O-Alkyl, COO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NarylAlkyl, NHCO-Aryl bedeuten, wobei Alkyl und Aryl die oben genannte Bedeutung haben, dabei können R⁷ und R⁸ auch durch eine kovalente Bindung verknüpft sein, so daß ein anneliertes Ringsystem vorliegt.

2. Verwendung von Verbindungen der Formel (I) nach Anspruch 1 dadurch gekennzeichnet, daß die Gruppe R³ gleich COOH, OH, SH, SO₃H, B(OH)₂, NHCOCH₃ oder PO(OH)₂ ist.

3. Verwendung von Verbindungen der Formel (I) nach einem der vorherigen Ansprüche dadurch gekennzeichnet, daß die Reste R⁴ bis R⁷, besonders bevorzugt Rest R⁴ und/oder R⁶, mindestens einen elektronenziehenden Substituenten, bevorzugt aus der Gruppe COO-Alkyl, Fluor, Chlor, Brom, lod, OH, NO₂, NO, CN, COOH, CHO, SO₃H, CF₃, CO-Alkyl, CO-Aryl, COO-Aryl, POAryl₂, SO₃-Alkyl enthalten.

4. Verfahren zur enantioselektiven Gewinnung von Aminosäuren oder Aminosäurederivaten aus deren Estern durch Umsetzung von Gemischen von Enantiomeren- und/oder Diastereomeren des Esters in Gegenwart eines Racemisierungskatalysators der Formel (I), 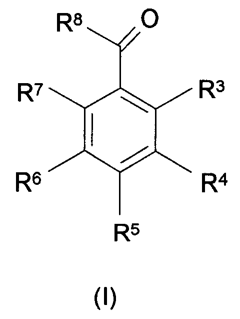 worin
R³ eine Gruppe mit einen aciden Proton,
R⁴-R⁷ unabhängig voneinander Wasserstoff, Alkyl, O-Alkyl, COO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NArylAlkyl, NHCO-Aryl und
R⁸ Wasserstoff, Alkyl oder Aryl ist, und
wobei Alkyl und Aryl die oben genannte Bedeutung haben, dabei können R⁷ und R⁸ auch durch eine kovalente Bindung verknüpft sein, so daß ein anneliertes Ringsystem vorliegt und in Gegenwart eines Enzyms zur enantioselektiven Esterspaltung.

5. Verfahren nach Anspruch 4 dadurch gekennzeichnet, daß die Umsetzung in einem homogenen Medium erfolgt.

6. Verfahren nach Anspruch 4 dadurch gekennzeichnet, daß die Umsetzung in einem heterogenen Medium erfolgt.

7. Verfahren nach Anspruch 6 dadurch gekennzeichnet, daß einer der Reaktionspartner bevorzugt das enantioselektiv esterspaltende Enzym auf einem Träger immobilisiert wird.

8. Verfahren nach einem der Ansprüche 4 bis 7 dadurch gekennzeichnet, daß eine enantioselektive Lipase, Esterase, Protease oder Alkoholase zugesetzt wird.

9. Verfahren nach Anspruch 8 dadurch gekennzeichnet, daß als enantioselektives Enzyme α-Chymotrypsin (EC 3.4.21.1 aus Rinderpankreas), Alcalase (EC 3.4.21.14 Protease von *bacillus licheniformis),* Subtilisin (EC 3.4.21.62 Protease von *Bacillus licheniformis);* Chirazym P1, ChiroClec® -BL oder eine Lipase aus Schweinepankreas (PPL, EC 3.1.1.3) eingesetzt wird.

10. Verfahren nach einem der Ansprüche 4 bis 9 dadurch gekennzeichnet, daß der Katalysator in einer Konzentration von 0,001 bis 20 mol% , bevorzugt in einer Konzentration von 0,1 bis 10 mol% bezogen auf das Substrat zugesetzt wird.

11. Verfahren nach einem der Ansprüche 4 bis 10 dadurch gekennzeichnet, daß die Umsetzung bei einer Temperatur von 0 - 100 °C, bevorzugt bei einer Temperatur von 20 - 60 °C durchgeführt wird.

12. Verfahren nach einem der Ansprüche 4 bis 11 dadurch gekennzeichnet, daß die Umsetzung in einem wäßrig-organischen Medium stattfindet.

13. Verfahren nach Anspruch 12 dadurch gekennzeichnet, daß die Umsetzung in Gegenwart von Cosolventien, insbesondere Dimethoxyethan, Aceton, Acetonitril, THF, Dioxan, Hexan, tert.-Butylmezhylether oder tert.-Butanol abläuft.

14. Verfahren nach einem der Ansprüche 4 bis 13 dadurch gekennzeichnet, daß die Umsetzung unter Zusatz eines Puffers, wie z. B. eines Phosphat- oder Tris-Puffers oder unter Zusatz einer Base, wie z. B. Natronlauge, bevorzugt in einem Bereich von pH 6,5 - pH 9,5 abläuft.

15. Verfahren nach einem der Ansprüche 4 bis 14 dadurch gekennzeichnet, daß als Edukt Verbindungen der allgemeinen Formel (II) 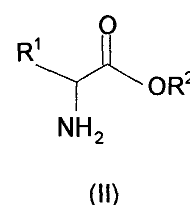 worin
R¹ und R² unabhängig voneinander für einen Alkyl-, Alkenyl-, Alkinyl-, Aryloder Heteroarylrest steht, eingesetzt, wobei Alkyl-, Alkenyl-, Alkinyl- eine aliphatische Kohlenstoffgruppe mit 1 bis 18 C-Atomen, die linearen, verzweigt und/oder auch cyclisch ist, stehen und Aryl einen 4 bis zu 14 C-Atome enthaltenden fünf-, sechsoder siebengliedrigen aromatischen Ring, wobei dieser Ring anneliert sein kann und 0 bis 3 Heteroatome wie N, O, S enthalten kann, bedeutet, dabei können sowohl die Alkyl- als auch die Arylgruppe gegebenenfalls bis zu sechs weitere Substituenten tragen, die unabhängig voneinander Wasserstoff, Alkyl, O-Alkyl, OCO-Alkyl, O-Aryl, Aryl, Fluor, Chlor, Brom, lod, OH, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, S-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂, PO(O-Aryl)₂, PO(O-Alkyl)(O-Aryl), PO(O-Alkyl)Alkyl, PO(O-Alkyl)Aryl, PO(O-Aryl)Alkyl, PO₂H-Alkyl, PO₂H-Aryl, PO(OH)Alkyl, PO(OH)Aryl SO₃-Alkyl, PO(O-Aryl)₂, PO(O-Alkyl)(O-Aryl), POArylAlkyl, SO-Aryl, SO₂-Aryl, SO₃-Aryl, N-Aryl₂, NH-Aryl, NArylAlkyl, NHCO-Aryl bedeuten und
R^{l} auch für CH₂CH₂POAryl₂, CH₂CH₂PO₃H₂, CH₂CH₂PO(O-Alkyl)₂, CH₂CH₂PO(O-Aryl)₂, CH₂CH₂PO(O-Alkyl)(O-Aryl), CH₂CH₂PO(O-Alkyl)Alkyl, CH₂CH₂PO(O-Alkyl)Aryl, CH₂CH₂PO(O-Aryl)Alkyl, CH₂CH₂PO₂H-Alkyl, CH₂CH₂PO₂H-Aryl, stehen kann, wobei Alkyl und Aryl die oben genannte Bedeutung haben. eingesetzt werden.

16. Verfahren nach einem der Ansprüche 4 bis 15 dadurch gekennzeichnet, daß der Reaktionsmischung ein Aminosäureester, bevorzugt mit einem Gewichtsanteil von 2 - 50 %, zugesetzt wird.
